# EUROPEAN PATENT SPECIFICATION

(11) **EP 3 600 541 B1**
(45) Date of publication and mention of the grant of the patent: **14.10.2026**
(21) Application number: 18771109.8
(22) Date of filing: 20.03.2018
(51) Int. Cl.: A61N 7/00, A61N 5/06, A61N 5/10, A61B 18/14, A61B 5/00, A61B 5/0205, A61B 5/0215, A61B 5/0245, A61B 5/02, A61B 5/029, A61B 5/055, A61B 5/087, A61B 5/107, A61B 8/08

(54) **SYSTEM FOR TREATING HEART FAILURE BY IMPROVING EJECTION FRACTION OF A PATIENT**
SYSTEM ZUR BEHANDLUNG VON HERZVERSAGEN DURCH VERBESSERUNG DER EJEKTIONSFRAKTION EINES PATIENTEN
SYSTÈME DE TRAITEMENT D'UNE INSUFFISANCE CARDIAQUE PAR AMÉLIORATION DE LA FRACTION D'ÉJECTION D'UN PATIENT

(30) Priority: 20.03.2017 US 201762473512 P; 20.03.2017 US 201762473545 P; 20.03.2017 US 201762473532 P
(43) Date of publication of application: 05.02.2020
(60) Divisional application: 26186843.4 / 4 799 684
(73) Proprietor: Sonivie Ltd., 4802126 Rosh Haayin (IL)
(72) Inventor: SHABTAY, Or, 2011000 Kibbutz Farod (IL); SHAV, Dalit, 7608654 Rehovot (IL)
(74) Representative: Vossius & Partner Patentanwälte Rechtsanwälte mbB
(86) International application number: PCT/IL2018/050316
(87) International publication number: WO 2018/173047

(56) References cited:
- WO-A1-2015/193889
- WO-A2-2016/084081
- US-A1- 2012 215 106
- US-A1- 2013 204 068
- US-A1- 2014 180 277
- US-A1- 2015 073 400
- US-A1- 2016 256 216
- US-A1- 2017 027 645
- US-B2- 8 715 209
- ZHANG, HANG ET AL.: "Pulmonary artery denervation for treatment of a patient with pulmonary hypertension secondary to left heart disease", PULMONARY CIRCULATION, vol. 6, no. 2, June 2016 (2016-06-01), pages 240 - 243, XP055610431
- CHEN, SHAO-LIANG ET AL.: "Percutaneous pulmonary artery denervation completely abolishes experimental pulmonary arterial hypertension in vivo", EUROLNTERVENTION, 8 March 2013 (2013-03-08), pages 269 - 76, XP055245268
- NATALE, ANDREA ET AL.: "The State of the Art Based on the VeniceChart International Consensus Document", 31 December 2009 (2009-12-31), pages 1 - 2, XP055625104, Retrieved from the Internet <URL:https://books.google.co.il/books?hl=iw&lr=&id=hmA8bZUzAHMC&oi=fnd&pg=PP2&ots=SM-yWpxSX6&sig=j5LlFWmM5Qop7mK527qg9-CgyEY&redir_esc=y#v=onepage&q&f=false>

## Description

### TECHNICAL FIELD

The present disclosure relates generally to the field of pulmonary artery denervation for improving a patient's ejection fraction.

### BACKGROUND

Ejection fraction refers to the percentage of blood that is pumped out of a filled ventricle with each heartbeat. Left ventricular ejection fraction is a measure of the efficiency of pumping into the systemic circulation, whereas right ventricular ejection fraction is a measure of the efficiency of pumping into the pulmonary circulation. The left ventricle is the heart's main pumping chamber that pumps oxygenated blood through the ascending (upward) aorta to the rest of the body, so ejection fraction is usually measured in the left ventricle (LV). It is calculated by dividing the stroke volume by the end-diastolic volume and is an inherent measure of the pumping efficiency of the heart. Hearts that beat busily but do not succeed in expelling much blood are said to be in heart failure.

A reduced ejection fraction may have multiple causes including cardiomyopathy, damage to the heart as a result of heart attack, heart valve malfunction, long-standing, uncontrolled high blood pressure, high pulmonary ventricle resistance, low distensibility of the arteries, and more.

Ejection fraction is commonly measured by an echocardiogram and serves as a general measure of a person's cardiac function. Other, rarer methods include cardiac catheterization, MRI, CT and nuclear methods such as PET, SPECT and MUGA, in which trace amounts of radioactive material - such as thallium - are injected into the bloodstream and special cameras detect the radioactive material in the blood as it flows through the heart and lungs.

The normal ejection fraction, which demonstrates good ventricular contractility, ranges between 50 to 75%. Reduced ejection fraction (less than 50%) indicates reduced heart pumping efficiency, where the heart does not manage to pump enough blood to the rest of the body and does not meet the body's needs. Patients having a reduced ejection fraction typically suffer from shortness of breath, inability to exercise, swelling of lower limbs, fatigue and weakness, mental confusion and heart arrhythmia. An ejection fraction of 35% or lower is a marker of an increased risk of heart failure, pulmonary congestion, hypertrophy or enlargement of the heart, as well as liver or kidney malfunction.

Document WO 2016/084081 A2 discloses a method for selectively modifying nerve activity without causing substantial damage to non-targeted tissue, comprising introducing a catheter device comprising one or more ultrasonic transceivers to the pulmonary artery lumen, receiving, using the one or more ultrasonic transceivers, echo signals reflected from the non-targeted tissue following emission of ultrasound energy by the one or more transceivers, analyzing the received echo signals to identify at least one of a type and location of the non-targeted tissue relative to the one or more transceivers, and emitting ultrasound energy from the one or more ultrasonic transceivers in accordance with the analysis, to modify nerve activity without substantially damaging the identified non-targeted tissue.

### SUMMARY

Aspects of the disclosure, in some embodiments thereof, relate to a system for improving an ejection fraction of a patient in need thereof.

A moderately reduced ejection fraction can be improved by so called life style changes including low salt diet, fluid management and exercise. However, often such life style changes are not enough. Certain medications can help reduce the heart's workload, increase blood flow, widen vessels or eliminate excess water from the body, all of which may help in treating low ejection fraction. Commonly prescribed medications include: Inotropes (such as digoxin), Angiotensin II receptor blockers and Betablockers. However, some patients do not respond to these medications, leaving cardiac defibrillator implantation as an alternative. There thus remains a need for minimally invasive systems for improving an ejection fraction of a patient.

The invention is defined in the following claims. Other embodiments, devices, examples and, in particular, therapeutic treatment methods are not a part of the invention and are described for illustrative purposes only.

### BRIEF DESCRIPTION OF THE DRAWINGS

Some embodiments of the disclosure are described herein with reference to the accompanying figures. The description, together with the figures, makes apparent to a person having ordinary skill in the art how some embodiments of the disclosure may be practiced. The figures are for illustrative purposes only.
**FIG. 1** is an illustrative flowchart of a non-claimed method for improving an ejection fraction of a patient, and
**FIG. 2** is an illustrative flowchart of the TROPHY 1 study described in Example 1 herein;
**FIG. 3A** shows average changes in mean Pulmonary Artery Pressure (mPAP) 4 months after the denervation procedure (Mean/Standard Err; Wilcoxon test 4M Vs Baseline; n = 11 (Patient 001-004 excluded));
**FIG. 3B** shows average changes in cardiac index 4 months after the denervation procedure (Mean/Standard Err; Wilcoxon test 4M Vs Baseline; n = 11 (Patient 001-004 excluded));
**FIG. 3C** shows average changes in pulmonary vascular resistance (PVR) 4 months after the denervation procedure (Mean/Standard Err; Wilcoxon test 4M Vs Baseline; n = 11 (Patient 001-004 excluded));
**FIG. 3D** shows average changes in right arterial pressure (RAP) 4 months after the denervation procedure (Mean/Standard Err; Wilcoxon test 4M Vs Baseline; n = 11 (Patient 001-004 excluded));
**FIG. 4A** shows average changes in quality-of-life (QoL) score, 4 months after the denervation procedure (Mean/Standard Err; Wilcoxon test 4M Vs Baseline; n = 11 (Patient 001-004 excluded));
**FIG. 4B** shows average changes in 6-minute walking distance (6MWD), 4 months after the denervation procedure (Mean/Standard Err; Wilcoxon test 4M Vs Baseline; n = 11 (Patient 001-004 excluded)); and
**FIG. 4C** shows average changes in daily activity (actimetry), 4 months after the denervation procedure (Mean/Standard Err; Wilcoxon test 4M Vs Baseline; n = 11 (Patient 001-004 excluded)).

### DETAILED DESCRIPTION

In the following description, various aspects of the disclosure will be described. For the purpose of explanation, specific configurations and details are set forth in order to provide a thorough understanding of the different aspects of the disclosure. However, it will also be apparent to one skilled in the art that the disclosure may be practiced without specific details being presented herein. Furthermore, well-known features may be omitted or simplified in order not to obscure the disclosure.

The present disclosure relates generally to the field of pulmonary artery denervation for improving an ejection fraction, in particular a left ventricular ejection fraction of a patient in need thereof.

The non-claimed method relates to positioning an artery manipulation device in or in proximity to a target blood vessel, e.g. within the pulmonary vasculature of a patient having a reduced ejection fraction and impairing the activity of at least one sympathetic nerve, nerve fiber or neuron, using the artery manipulation device. The impairment of the nerve, nerve fiber or neuron leads to denervation of the target blood vessel and, as a result thereof, to an improvement in the ejection fraction.

Parasympathetic nerves of the pulmonary artery are treated. According to some embodiments, nerves may be treated at a neuron-by-neuron level. According to some embodiments, whole nerves may be treated. For the purposes of this disclosure, the phrase "nerves or neurons" may be used to treat one or more whole nerves, one or more nerve fibers, and/or one or more neurons.

This process of partial or complete nerve destruction may be referred to herein as "denervating" or "denervation of a structure. For example, in some embodiments, the therapy may involve denervating one or more arteries supplying the lungs, such as, but not limited to, the pulmonary trunk, the left pulmonary artery and/or the right pulmonary artery.

As used herein the term "artery manipulation device" is treatment device configured to cause pulmonary artery denervation.

The manipulation device includes a catheter and an ultrasound emitter mounted on the catheter. The ultrasound beam **is** unfocused ultrasound beam. The ultrasound beam is emitted at a frequency of 8-12 MHz.

The catheter has a length of 100-150 cm, such as, but not limited to, about 120 cm.

A duration of treatment at each site ranges between, 20-70 seconds.

The applied ultrasound intensity ranges between 30-70 W/cm^2, such as 40W/cm^2, 50 W/cm^2, 60W/cm^2

In some embodiments, one or more of the following safety measures are taken before, after and/or during treatment: in some embodiments, the catheter is used for treating a single patient only; in some embodiments, re-sterilization and/or re-use of the catheter is avoided; in some embodiments, the catheter is used only with its compatible console; in some exemplary, non-claimed embodiments, the method is performed while avoiding applying direct pressure onto the distancing device and/or ultrasonic transducers of the device during preparation; in some embodiments, during treatment, if the distancing device is open, manipulation and/or retraction of the catheter are avoided; in some embodiments, during energy delivery, the catheter is not moved; in some embodiments, for example in cases in which additional electrical devices are used during operation in addition to the catheter, the system is disconnected by unplugging the power cable and/or by withdrawing the catheter; in some embodiments, in the event the distancing device malfunctions, the distancing device can be retracted along with the catheter and/or along with the guide sheath; in some cases in which fluoroscopy is performed, care should be taken to avoid excessive exposure of the patient to contrast agents; in some embodiments, before treatment, a patient is provided with a systemic anticoagulant. Optionally, an activated clotting time (ACT) is monitored during treatment. In some cases, an ACT of at least 250 seconds should be maintained. In some embodiments, use of the catheter and/or console is allowed only for trained, qualified medical personnel (e.g. right heart catheterization experts); in some embodiments, during the procedure, Echocardiography and equipment for diagnosis and immediate therapeutic actions for pericardial tamponade should be made available; in some embodiments, care should be taken to avoid using electro-medical energy sources in the presence of flammable detergents, anesthetics, nitrous oxide (N2O), or oxygen that are not controlled in a closed-circuit environment; in some embodiments, use of the system is permitted only following completion of training; in some cases in which tissue dissection or perforation of the pulmonary artery have occurred, care should be taken when introducing the catheter; in some embodiments, the catheter and/or other accessories (e.g. distancing device) are to be disposed of according to established hospital protocols of biohazardous waste disposal; in some embodiments, only predefined intended target areas are treated; in some embodiments, powered and/or automatic contrast injection devices may be used before and/or during the procedure; optionally, specified settings for flow injection and/or contrast agent delivery are taken into consideration; in some embodiments, water ingress into the console should be avoided; in some cases, if the patient suffers from excessive pain during treatment, analgesia and/or sedation should be considered; in some cases, if radiography is used, radiation hazards should be handled.

According to some exemplary embodiments, the method further comprises indicating one or more side effect on the instruction to use. According to some embodiments, the side effects may be selected from the group consisting of: heart rhythm disturbances including bradycardia; formation of blood clot and/or embolism, possibly resulting in ischemic events such as myocardial infarction, stroke, hemoptysis; hematoma, bruising, bleeding; vascular complications including arterial spasm, arterial stenosis, arterial dissection, perforation, pulmonary perforation, pseudo-aneurysm, AV-fistula; complications associated with the contrast agent used during the procedure, e.g., serious allergic reaction or reduced kidney function; pain; infection; nausea or vomiting; fever; death; cardiopulmonary arrest; risks associated with contrast agents, narcotics, anxiolytics, other pain medications and anti-vasospasm agents used during the procedure; biological hazards: risks of infection, toxicity, adverse hematology, allergy, hemorrhage, hemoptysis, pain and pyrogenicity; damage to the blood vessel wall or other body structures from the delivery of energy, e.g., pulmonary artery stenosis, nerve damage, spasm, aneurysm formation or rupture; unintended/unexpected damage to local anatomical structures such as the cardiac plexus, esophagus, trachea, lung or vagus, leading to bradycardia, tachycardia, partial vocal cord palsy or Haemoptysis; decreased pulmonary function; hypertension; hypo-perfusion, or any combination thereof. Each possibility is a separate embodiment.

As referred to herein, the terms "patient" and "subject" may interchangeably be used and may relate to a subject having a reduced ejection fraction.

As used herein, the term "ejection fraction" refers to a percentage of blood that is pumped out of a filled ventricle with each heartbeat. According to some embodiments, the ejection fraction may be left ventricular ejection fraction. According to some embodiments, the ejection fraction may be right ventricular ejection fraction.

According to some embodiments, a reduced ejection fraction may be defined as an ejection fraction below 55 percent, below 50 percent, or below 35 percent. Each possibility is a separate embodiment.

According to some embodiments, the patient having a reduced ejection fraction may include patients with an increased trans-pulmonary gradient (TPG) and/or an elevated pulmonary vascular resistance (PVR). As used herein, the term "trans-pulmonary gradient" may refer to the difference between mean pulmonary arterial pressure (mPAP) and left atrial pressure (PLA; commonly estimated by pulmonary capillary wedge pressure: PCWP). According to some embodiments, an increased TPG may refer to an mPAP-PCWP that exceeds 12-15 mmHg. According to some embodiments, an elevated PVR may refer to a PVR above 2 Wood units, above 2.5 Wood units, above 3 Wood units or above 3.5 Wood units.

According to some embodiments, improving the ejection fraction may include increasing an ejection fraction by 5 percent, or by 10 percent.

According to some embodiments, improving the ejection fraction may include decreasing the pulmonary vascular resistance (PVR) in at least one artery of the pulmonary vasculature.

According to some embodiments, the PVR of the patient is at least 2.5 Wood units. According to some embodiments, the PVR of the patient remains essentially unchanged when a pulmonary capillary wedge pressure (PCWP) of the patient is reduced. According to some embodiments, the PVR of the patient remains essentially unchanged when the patient is treated with a diuretic and/or a vasodilator.

According to some embodiments, improving an ejection fraction of the patient comprises an increase in the patient's cardiac index. As used herein, the term "cardiac index" and "CI" are interchangeable and refer to the hemodynamic parameter that relates the cardiac output (CO) from left ventricle in one minute to body surface area (BSA), thus relating heart performance to the size of the individual. The unit of measurement is liters per minute per square meter (L/min/m2). The normal range of cardiac index at rest is 2.6-4.2 L/min/m2.

According to some embodiments, improving an ejection fraction of the patient comprises an increase in the patient's right atrial pressure. As used herein, the terms "right atrial pressure", "RA Pressure" and "RAP" may interchangeably refer to the blood pressure in the right atrium of the heart. The RAP reflects the amount of blood returning to the heart and the ability of the heart to pump the blood into the arterial system. The normal range of RAP is 2 - 6 mmHg or below 8 mmHg.

According to some embodiments, improving an ejection fraction of the patient results in an improvement in quality of life (QoL) parameters. Non-limiting examples of suitable QoL parameters include: quality of life (QoL) score (the patient's subjective assessment of his/her quality of life); 6-minute walking distance (6MWD), actimetry (daily activity).

According to some embodiments, the herein disclosed denervation treatment may improve proximal pulmonary arteries' compliance which in turn may reduce right ventricular afterload.

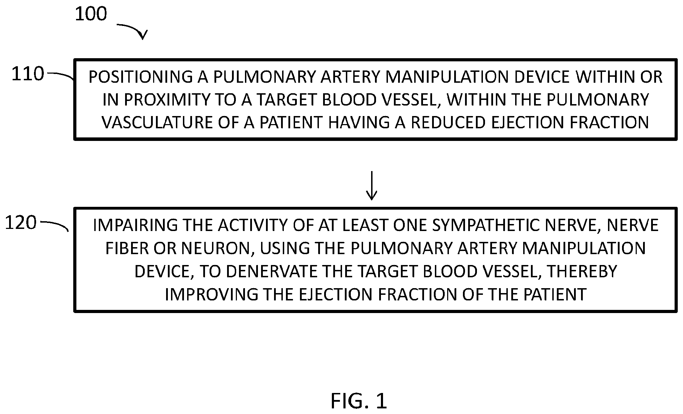

Reference is now made to **FIG. 1** which is an illustrative flowchart of a method **100** for improving an ejection fraction of a patient, In step **110** a pulmonary artery manipulation device is positioned in a target blood vessel, within the pulmonary vasculature of a patient having a reduced ejection fraction. In step **120** the activity of at least one sympathetic nerve is impaired in order to denervate the target blood vessel and thereby improve the ejection fraction of the patient.

### EXAMPLES

### Example 1 - Pulmonary artery denervation improves ejection fraction in human study (TROPHY 1)

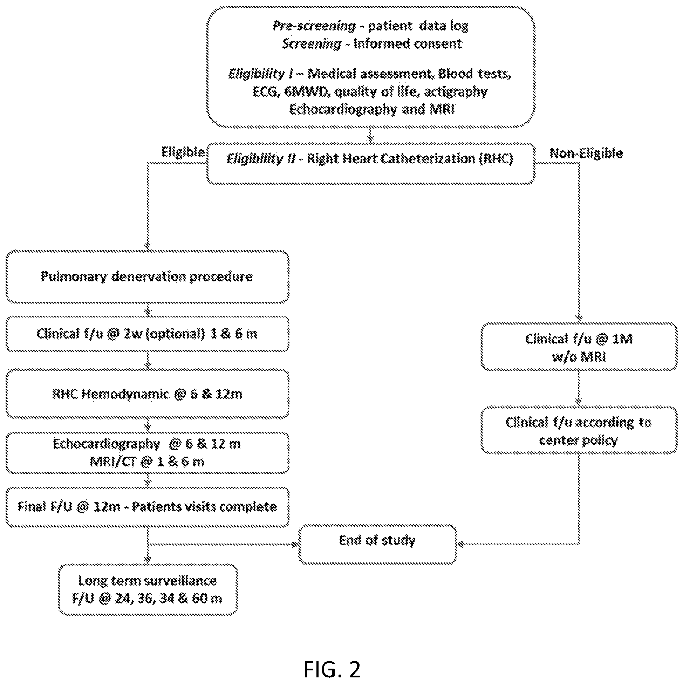

The TROPHY 1 study is a clinical evaluation of the Therapeutic Intra-Vascular Ultrasound (TIVUS^{™}) System for pulmonary artery denervation in patients with reduced ejection fraction. The outline of the study is shown in the flowchart of **FIG. 2****.** 15 patients were enrolled in the study, and initial results have been obtained for three patients. The study was aimed at assessing the safety, performance and initial effectiveness of pulmonary denervation on ejection fraction directly and through the evaluation of heart function, exercise tolerance (6 minutes walking distance) and quality of life questionnaires. The pulmonary denervation was performed (in patients eligible according to study criteria), in the right, left and main pulmonary arteries according to manufacturer's instructions. The duration of the denervation procedure was 20-30 minutes on average, and the usability feedback from the operators was excellent. The number of denervation sites for each patient varied between 6 and 16, according to the specific patient anatomy. Following the procedure, patients were evaluated, according to the study protocol, 1, 4, 8 and 12 months post procedure. Patient ejection fraction was measured using MRI before and 1 and 12 months after the procedure. Initial results (n=3), from the 1 month follow-up, demonstrate an average increase of 28% in the ejection fraction in both right and left ventricles following the pulmonary denervation, as set forth in table 1 below.

**Table 1 - MRI based ejection fraction before and 1 month after pulmonary denervation**

| **Patient** | **Ejection Fraction Pre-Procedure (%)** | **Ejection Fraction 1M Post-Procedure (%)** | **LV/RV** |
|---|---|---|---|
| 002-001 | 23 | 31 | RV |
| 001-001 | 11 | 16 | RV |
| 001-001 | 48 | 60 | LV |
| 001-002 | 48 | 52 | RV |

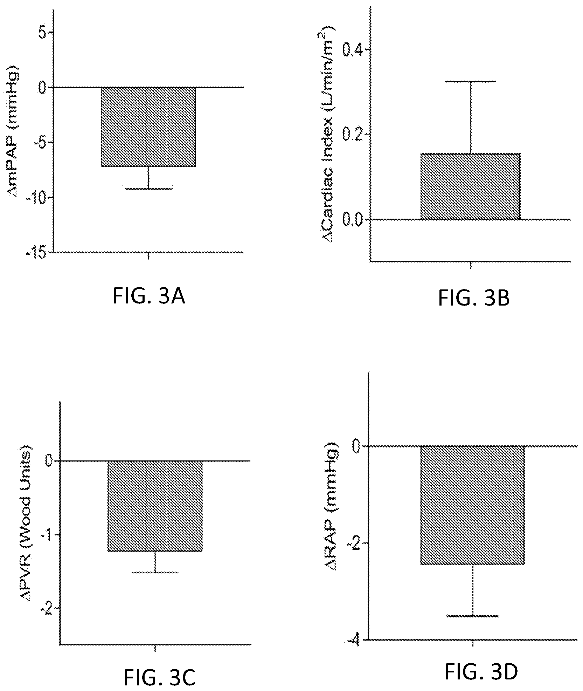

At the 4-month follow-up, all subjective and objective measurable parameters were improved. **FIG. 3A****-****FIG. 3D** shows average changes in hemodynamic parameters 4 months after the denervation procedure (Mean/Standard Err; Wilcoxon test 4M Vs Baseline; n = 11 (Patient 001-004 excluded)).

Advantageously, the mean Pulmonary Artery Pressure (mPAP) was reduced by 13.5% (mean ± sd = -7.2 ± 6.6, p = 0.010), as shown in **FIG. 3A****;** the cardiac index increased by 2% (mean ± sd = 0.15 ± 0.52, p = 0.695), as seen in **FIG. 3B****;** pulmonary vascular resistance (PVR) reduced by 15.0% (mean ± sd = -1.32 ± 0.98, p = 0.003), as seen in **FIG. 3C****;** and the right arterial pressure reduced by 20.5% (mean ± sd = -2.45 ± 3.5, p = 0.053), as seen in **FIG. 3D****.** These results suggest that Therapeutic Intra-Vascular Ultrasound treatment can improve proximal Pulmonary Arteries compliance which in turn reduces right ventricular afterload.

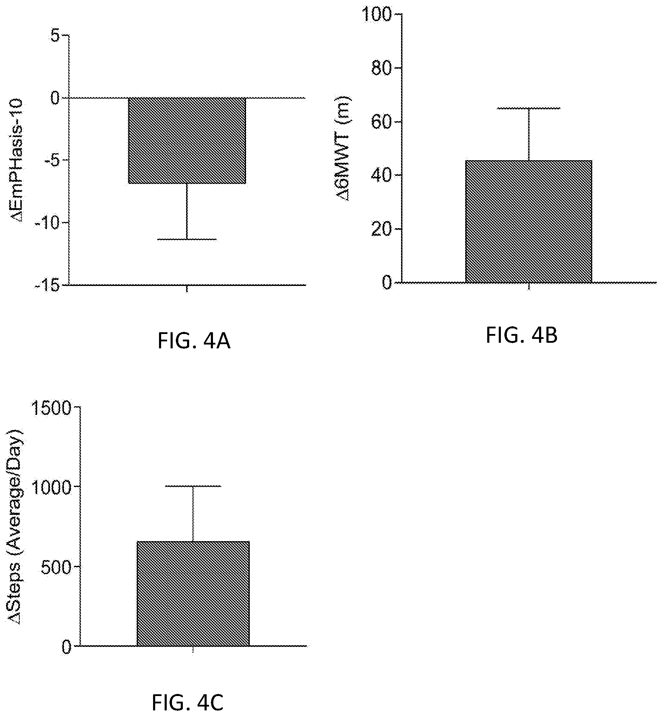

**FIG. 4A****-****FIG. 4C** shows average changes in quality-of-life (QoL) parameters, 4 months after the denervation procedure (Mean/Standard Err; Wilcoxon test 4M Vs Baseline; n = 11 (Patient 001-004 excluded)).

Advantageously, a positive change in the quality of life score (QoL) indicated by a 21% reduction in the EmPHasis-10 was observed (mean ± sd = -6.9 ± 14, p = 0.275), as shown in **FIG. 4A****.** Moreover, a 40 meter (21%) improvement in the 6-minute walking distance (6MWD) was like wise observed (mean ± sd = 45.5 ± 64.6, p = 0.065), as seen from **FIG. 4B****,** as well as a 12% increase in daily activity (actimetry) (mean ± sd = 584 ± 883, p = 0.141) as seen from **FIG. 4C****.**

The examples described above are non-limiting examples and are not intended to limit the scope of the disclosure. The described examples may comprise different features, not all of which are required in all embodiments of the disclosure.

The terminology used herein is for the purpose of describing particular embodiments only and is not intended to be limiting. As used herein, the singular forms "a", "an" and "the" are intended to include the plural forms as well, unless the context clearly indicates otherwise. It will be further understood that the terms "comprises" or "comprising", when used in this specification, specify the presence of stated features, integers, steps, operations, elements, or components, but do not preclude or rule out the presence or addition of one or more other features, integers, steps, operations, elements, components, or groups thereof.

In the description and claims of the application, each of the words "comprise" "include" and "have", and forms thereof, are not necessarily limited to members in a list with which the words may be associated.

## Claims

1. A system for improving an ejection fraction of a patient in need thereof and having a pulmonary vascular resistance (PVR) of at least 3 Wood units, the system being configured to cause pulmonary artery denervation and the system comprising:
- an intravascular treatment device comprising a catheter having a length of 100-150 cm, the device comprising an ultrasound transmitter mounted on the catheter and arranged for positioning within a target pulmonary blood vessel,
- a compatible console for the catheter,
the system configured to emit, via the ultrasound transmitter, unfocused ultrasound energy at a frequency of 8-12 MHz and an intensity of 30-70 W/cm² for 20-70 seconds to thereby denervate at least one parasympathetic nerve of the target pulmonary blood vessel, thereby improving the ejection fraction of the patient.

## Patentansprüche

1. System zur Verbesserung einer Ejektionsfraktion eines Patienten, der dies benötigt und einen pulmonalen Gefäßwiderstand (PVR) von mindestens 3 Wood-Einheiten hat, wobei das System so konfiguriert ist, dass es pulmonale Arteriendenervation bewirkt, und das System aufweist:
- eine intravaskuläre Behandlungsvorrichtung, die einen Katheter mit einer Länge von 100 bis 150 cm aufweist, wobei die Vorrichtung einen Ultraschallsender aufweist, der an dem Katheter angebracht und zur Positionierung in einem pulmonalen Zielblutgefäß ausgebildet ist,
- eine kompatible Konsole für den Katheter,
wobei das System so konfiguriert ist, dass es über den Ultraschallsender unfokussierte Ultraschallenergie mit einer Frequenz von 8 bis 12 MHz und einer Intensität von 30 bis 70 W/cm² für 20 bis 70 Sekunden abgibt, um dadurch mindestens einen parasympathischen Nerv des pulmonalen Zielblutgefäßes zu denervieren und damit die Ejektionsfraktion des Patienten zu verbessern.

## Revendications

1. Système destiné à améliorer une fraction d'éjection d'un patient qui en a besoin et ayant une résistance vasculaire pulmonaire (RVP) d'au moins 3 unités Wood, le système étant configuré pour induire une dénervation d'artère pulmonaire et le système comprenant:
- un dispositif de traitement intravasculaire comprenant un cathéter ayant une longueur de 100 à 150 cm, le dispositif comprenant un émetteur d'ultrasons monté sur le cathéter et agencé pour un positionnement dans un vaisseau sanguin pulmonaire cible,
- une console compatible pour le cathéter,
le système étant configuré pour émettre, via l'émetteur d'ultrasons, une énergie ultrasonore non focalisée à une fréquence de 8 à 12 MHz et une intensité de 30 à 70 W/cm² pendant 20 à 70 secondes pour dénerver ainsi au moins un nerf parasympathique du vaisseau sanguin pulmonaire cible, améliorant ainsi la fraction d'éjection du patient.
